# EUROPÄISCHE PATENTSCHRIFT

(11) **EP 0 541 003 B1**
(45) Veröffentlichungstag und Bekanntmachung des Hinweises auf die Patenterteilung: **20.01.1999**
(21) Anmeldenummer: 92118483.4
(22) Anmeldetag: 29.10.1992
(51) Int. Cl.: C07D 473/18

(54) **Verfahren zur Herstellung von Guanin und seiner Alkalimetallsalze**
Process for the preparation of guanine and its alkali metal salts
Procédé pour la préparation de la guanine et de ses sels de métaux alcalins

(30) Priorität: 02.11.1991 DE 4136114
(43) Veröffentlichungstag der Anmeldung: 12.05.1993
(73) Patentinhaber: Boehringer Ingelheim Pharma KG, 55216 Ingelheim am Rhein (DE); BOEHRINGER INGELHEIM INTERNATIONAL GmbH, 55218 Ingelheim am Rhein (DE)
(72) Erfinder: Ramert, Reiner, Dr., W-6531 Weiler bei Bingen (DE); Müller, Manfred, Dr., W-6101 Bickenbach (DE); Bauer, Klaus, W-6501 Schwabenheim (DE)
(74) Vertreter: Laudien, Dieter, Dr.

(56) Entgegenhaltungen:
- EP-A- 304 004
- EP-A- 415 028
- DE-A- 3 235 372
- DE-A- 3 723 874
- DE-A- 3 729 471
- CHEMICAL ABSTRACTS, vol. 109, no. 9, 29. August 1988, Columbus, Ohio, US; abstract no. 73470g, Seite 697 ;Spalte L ;
- JOURNAL OF THE AMERICAN CHEMICAL SOCIETY Bd. 75, Nr. 2, 29. Januar 1953, WASHINGTON Seiten 263 - 266 ROLAND K. ROBBINS ET. AL.
- PATENT ABSTRACTS OF JAPAN vol. 12, no. 365 (C-532)(3212) 29. September 1988
- PATENT ABSTRACTS OF JAPAN vol. 8, no. 102 (C-222)(1539) 12. Mai 1984
- PATENT ABSTRACTS OF JAPAN vol. 8, no. 114 (C-225)1984

## Beschreibung

Die Erfindung betrifft ein verbessertes Verfahren zur Herstellung von 2-Amino-1,9-dihydropurin-6-on bzw. seiner Alkalimetallsalze.

2-Amino-1,9-dihydro-purin-6-on (Guanin) ist ein interessantes Zwischenprodukt zur Synthese wichtiger pharmakologisch aktiver Verbindungen, von dem jährlich weltweit große Mengen benötigt werden. Aus diesem Grund befassen sich in den letzten Jahren mehrere Patentanmeldungen mit Verfahrensverbesserungen zur Herstellung von Guanin im technischen Maßstab, so z.B. die Europäische Patentanmeldung 304 004, die ein Eintopfverfahren" im technischen Maßstab offenbart. Aus der Europäischen Patentanmeldung 415 028 ist bekannt, daß 2,4,5-Triamino-6-hydroxy-pyrimidin-sulfat mit Alkaliformiat in Ameisensäure umgesetzt wird. Einen weiteren Zugang zu Purinderivaten eröffnet gemäß J.Am.Chem.Soc.Bd.75,1953,S.263-266 die Umsetzung von 5-Formylamino-pyrimidin-Derivaten mit Formamid. Dieses im Labormaßstab durchgeführte Verfahren läßt sich aufgrund der ungünstigen Mengenverhältnisse von Pyrimidin-Derivat zu Formamid nicht in wirtschaftlich sinnvoller Weise im technischen Maßstab durchführen.

Es ist vornehmlich die Aufgabe der vorliegenden Erfindung ein technisches Verfahren zur Verfügung zu stellen, das nicht nur ein reines Guanin liefert, sondern das auch mit einem höheren Durchsatz (kg/h) bzw. einer höheren Raum-Zeit-Ausbeute (kg/h.ltr) die Herstellung von Guanin ermöglicht.

Das erfindungsggemäße Verfahren ist dadurch charakterisiert, daß man 2,4-Diamino-5-formylamino-6-hydroxypyrimidin (FTAHP) bei einer Temperatur von mindestens 140°C in die mindestens doppelte Menge Formamid einträgt, das Reaktionsgemisch dann für 1 bis 5 Stunden bei einer Temperatur von mindestens 140° aber nicht mehr als 250°C cyclisiert, bevorzugt 185 - 195°C, anschließend abkühlt und das ausgefallene Guanin nach üblichen Methoden isoliert.

Die Reaktion kann drucklos oder unter Überdruck diskontinuierlich oder kontinuierlich durchgeführt werden.

Im Gegensatz zu den bisher bekannten Verfahren zur Synthese von Guanin kommt das erfindungsgemäße Verfahren mit wesentlich geringerer Menge an Lösungsmittel aus. Üblicherweise beträgt das Verhältnis von Ausgangsprodukt zu Lösungsmittel 1 : 5 (Beispiel 1 EP 415 028), 1 : 6 (DOS 37 29 471, Beispiel 1) bis 1 : 16 (Pfleiderer in Liebigs Ann. Chem. 647, 173, W.Traube, Berichte 33, 1371) und ist damit wesentlich ungünstiger als bei dem erfindungsgemäßen Verfahren. Ein aus dem erfindungsgemäßen Verfahren resultierender Vorteil ist, daß die Vorbereitungs- und Aufarbeitungszeiten infolge der verringerten Lösungsmittelmenge wesentlich verkürzt sind. Aus dem Stand der Technik werden Reaktionszeiten von mindestens 10 Stunden - bevorzugt sogar 16 - 25 Stunden (EP 415 028) angegeben. Das erfindunsgemäße Verfahren erfordert lediglich 1 bis 5 Stunden (technischer Maßstab) und erhöht damit eindeutig bei vorgegebener Apparaturgröße die Raum-Zeit-Ausbeute.

Syntheseverfahren zur Herstellung von Guanin unter Verwendung von Formamid sind im Prinzip aus dem Stand der Technik bekannt (z.B. DOS 37 29 471), wiesen bislang jedoch einen erheblichen Nachteil auf. So wird in der Europäischen Patentanmeldung 415 028 auf Seite 2, Zeile 21 ff. dazu ausgeführt, daß zur Herstellung eines farblich ansprechenden Guanins beispielsweise mindestens zwei Reinigungsfällungen und zwei Aktivkohlebehandlungen mit jeweils 50 Gew.-% Aktivkohle, bezogen auf Guanin, durchgeführt werden müssen.

Werden erfindungsgemäß die in Patentanspruch 1 aufgeführten Reaktionsbedingungen eingehalten, so erhält man ein Rohguanin, das ohne zusätzliche Reinigungsfällung durch drei einfache Kohlungen in alkalischer Lösung mit jeweils nur ca. 5 - 10 %-Gew.-Aktivkohle, bezogen auf Guanin, in ein hochreines Produkt überführt werden kann.

Ein überraschendes Ergebnis, das in Kenntnis der bekannten thermischen Labilität von Formamid (Zersetzung in Ammoniak und Kohlenmonoxid bzw. Wasser und Blausäure, wobei letztere zu braunen bis schwarzen Produkten polymerisiert) nicht zu erwarten war. Erfindungsgemäß ist es nicht erforderlich, daß das eingesetzte 2,4-Diamino-5-formylamino-6-hydroxypyrimidin wasserfrei ist. Ein Wassergehalt bis zu 50 % wirkt sich nicht wesentlich auf die Produktqualität aus (sofern Ameisensäure zugesetzt wird), verlängert aber die Cyclisierungszeit, da zur Aufrechterhaltung der Reaktionstemperatur Wasser abdestilliert werden muß. Schleuderfeuchtes FTAHP enthält in der Regel 20 - 50 Gew. % Wasser, kann also ohne Trocknung direkt weiter umgesetzt werden. Die Herstellung von FTAHP ist beispielsweise in der Europäischen Patentanmeldung 267 594 Beispiel 6/7) beschrieben, auf die hiermit inhaltlich Bezug genommen wird.

In einer bevorzugten Ausführungsform der Erfindung werden der Reaktionsmischung bis zu 10 %, besonders bevorzugt 2 - 3 % Ameisensäure und bis zu 5 %, bevorzugt 1 - 2 % Wasser zugesetzt. Der alleinige Zusatz von Wasser wirkt sich nachteilig auf die Produktqualität aus.

Bei der Cyclisierung von trockenem FTAHP hat es sich erwiesen, daß die Verwendung von technischer Ameisensäure 50 - 95 %ig, bevorzugt 85 %ig zu einem qualitativ besseren Guanin führt, als die Verwendung von reiner, d.h. 98 - 100 %iger Ameisensäure.

Eine technisch sinnvolle unterste Grenze für das Verhältnis von eingesetztem 2,4-Diamino-5-formylamino-6-hydroxypyrimidin und Formamid ist 1 : 2 (Gewichtsteile), da bei einem geringeren Anteil an Formamid das Reaktionsgemisch sich so verfestigt, daß ein Rühren unmöglich wird.

Ein optimales Verhältnis von 1 : 2,8 bis 1 : 3 ist bevorzugt und führt zu einem gut isolierbaren Guanin. Ein größerer Überschuß an Formamid ist natürlich denkbar, z.B. 1 : 7, führt aber von der erfindungsgemäßen gestellten Aufgabe eine möglichst hohe Raum-Zeit-Ausbeute zu erzielen, weg. Die Aufarbeitung des aus der Reaktionsmischung ausfallenden Guanins ist aus dem Stand der Technik bekannt und muß nicht näher erörtert werden. Bevorzugt ist eine alkalische Kohlung, bei der das Guanin in wässeriger Lauge (z.B. NaOH) gelöst, ein oder mehrfach gekohlt und anschließend mit Säure, z.B. Schwefelsäure, Kohlensäure (DE 40 04618) oder durch Verseifungsfällung mit Essig- oder Ameisensäureestern (DE 37 23 874), ausgefällt wird.

In einer Verfahrensvariante wird die bei der Cyclisierung von 2,4-Diamino-5-formylamino-6-hydroxypyrimidin in Formamid erhaltene Suspension auf Temperaturen um 100° C abgekühlt, mit ca. demselben Volumen Wasser versetzt, dann auf ca. 20° C abgekühlt. Anschließend wird mit Natronlauge (z.B. technische Natronlauge) eine Alkalikonzentration von mindestens 10 Gew.-% bezogen auf das Gesamtgewicht der Reaktionsmasse eingestellt. Das dabei erhaltene grobkristalline Salz kann besser abgeschleudert werden als das feinkristalliner ausfallende Guanin. Zur weiteren Reinigung kann das Natriumsalz in Wasser gelöst, mit Aktivkohle behandelt und anschließend mit Säuren - wie zuvor beschrieben - in Guanin überführt werden.

Wie bereits erwähnt, findet bei höheren Temperaturen eine thermische Zersetzung von Formamid zu Ammoniak und Kohlenmonoxid statt, die bei dem erfindungsgemäßen Verfahren etwa 1 Mol pro Mol Guanin, also ca. 300 g Gase pro kg Guanin beträgt. Dies ist technisch durch saure Gaswäsche und Abfackeln des nicht auswaschbaren Kohlenmonoxids beherrschbar. Andererseits ist auch eine Rückführung des Gasgemisches in die Hochdrucksynthese von Formamid (vergl. Ullmanns Encyclopedia of Industrial Chemistry, Vol. A12, p. 1 [1989]) denkbar. Des weiteren legen diese Erfahrungen auch ein Arbeiten unter Überdruck nahe, womit einerseits die Reaktionstemperatur weiter gesteigert, also die Reaktionszeit weiter verkürzt und andererseits die Zersetzung des Formamids verringert bzw. ganz unterdrückt werden kann. Aus diesem Grund kann die Cyclisierung unter Druck bei Temperaturen von 190 bis max. 250°C, und - technisch besonders interessant - in einer kontinuierlich arbeitenden Apparatur, durchgeführt werden.

Das erfindungsgemäße Verfahren zur Cyclisierung von 2,4-Diamino-5-formylamino-6-hydroxypyrimidin zu Guanin vermeidet den Einsatz zusätzlicher anorganischer Hilfschemikalien und die Verwendung von Lösunsmittelgemischen. Aufgrund dieser Tatsache kann das eingesetzte Lösungsmittel (Formamid) nach einer einfachen Redestillation wieder in das Verfahren eingesetzt werden. Das erfindungsgemäße Verfahren kann auf andere Purine, wie beispielsweise Xanthin, 2-Thioxanthin, 2-Aminopurin oder Adenin analog übertragen werden, indem die entsprechend substituierten Formyl-Vorstufen eingesetzt werden.

Die nachfolgenden Beispiele sollen das erfindungsgemäße Verfahren erläutern, ohne es jedoch auf diese Beispiele zu beschränken.

### Beispiel 1

### Cyclisierung von schleuderfeuchtem FTAHP unter minimalem Lösungsmitteleinsatz

203 g (1 mol) schleuderfeuchtes FTAHP mit einem Wassergehalt von 20 % werden in 250 ml Formamid unter Zusatz von 4,5 ml Ameisensäure bei Raumtemperatur suspendiert. In einer 75o ml Rührapparatur mit Destillationseinrichtung werden 175 ml Formamid auf 180°C erhitzt und unter weiterem Heizen die FTAHP-Suspension mit Hilfe einer Schlauchpumpe im Verlauf von 75 min. zudosiert, wobei die Sumpftemperatur nicht unter 175°C sinken darf. Nach 3 h Rühren bei 175 - 180°C wird die Suspension abgekühlt, bei 60°C abgesaugt, gründlich mit Wasser und Methanol gewaschen und getrocknet. Es werden 150,1 g (99,3 %) Guanin mit einem Gehalt (HPLC) von 97,9 % erhalten.

### Beispiel 2

### Cyclisierung von FTAHP im Technikumsmaßstab mit Isolierung von Guanin

In einem 250 l Email-Rührwerksapparat werden 105 l Formamid, 2,4 l 85 %ige Ameisensäure und 1,2 l Wasser vorgelegt und auf 180°C erhitzt. Über einen Feststoffdosiertrichter werden im Verlauf von ca. 30 min 42,3 kg FTAHP eingetragen. Hierbei soll die Sumpftemperatur nicht unter 175°C absinken. Anschließend wird noch 3 h bei 175 - 180° C gerührt, auf 90°C abgekühlt und abgeschleudert. Der Schleuderkuchen wird mit insgesamt 140 l Methanol gewaschen und bei 60°C im Umluftschrank getrocknet.

Es werden ca. 36 kg (95,2 %) Guanin mit einem Gehalt (HPLC) von 95 % erhalten. Die Schleuderlaugen werden in einem 300 l Email-Rührwerksapparat bei Normaldruck bis zu einer Sumpftemperatur von 100° C destilliert, wobei ca. 120 l Methanol übergehen. Anschließend wird im Vakuum weiterdestilliert. Die bei 90 - 100°C Kopftemperatur bei 10 - 15 mbar übergehende Fraktion, ca. 85 l, ist für den Wiedereinsatz geeignetes Formamid.

### Beispiel 3

### Cyclisierung von FTAHP im Technikumsmaßstab mit Isolierung von Dinatriumguanat

In einem 250 l Email-Rührwerksapparat werden 105 l aus vorhergegangenen Ansätzen gemäß Beispiel 2 zurückgewonnenes Formamid vorgelegt, auf 180°C erhitzt und, wie in Beispiel 2 beschrieben, 42,3 kg FTAHP eingetragen. Nach 3 h Reaktionszeit bei 175 - 180°C wird auf 100°C abgekühlt und unter weiterer Kühlung mit 125 l Wasser verdünnt. Nach Abkühlen auf unter 20°C werden insgesamt 50 l konz. techn. Natronlauge langsam zudosiert. Hierbei entsteht vorübergehend eine klare Lösung, aus der alsbald das Natriumsalz des Guanins auskristallisiert. Die Kristallisation wird bei + 5°C vervollständigt. Das grobkristalline Salz wird abgeschleudert, mit insgesamt 50 l eiskalter 15 %iger Natronlaue und 50 l Aceton gewaschen und im Umluftschrank bei 50 - 60°C getrocknet. Es werden ca. 65 kg kristallwasserhaltiges Dinatriumguanat mit einem Guaningehalt (HPLC) von ca. 53 % erhalten. Dies entspricht einer Ausbeute von 91,1 % d.Th. Das isolierte Dinatriumsalz kann in Reinguanin überführt werden, indem man es in Wasser löst, mit Aktivkohle behandelt und anschließend mit verdünnter Schwefelsäure zu Guanin hydrolysiert.

### Beispiel 4

### Herstellung von Reinguanin aus Rohguanin

70 kg des gemäß Beispiel 2 gewonnenen Rohguanins werden in 350 l Kondensat suspendiert und nach Zusatz von 55 l techn. konz. Natronlauge unter leichtem Erwärmen gelöst. Nach Zugabe von 7 kg Aktivkohle wird noch 20 Minuten gerührt und dann die Kohle über ein Mehrschichtenfilter abgetrennt. Diese Kohlebehandlung wird zweimal mit je 7 kg Aktivkohle wiederholt. Die gereinigte Lösung wird in bekannter Weise bei erhöhter Temperatur mit verdünnter Schwefelsäure neutralisiert. Das ausgefällte Reinguanin wird abgeschleudert, gründlich mit Wasser und Methanol gewaschen und zur Gewichtskonstanz getrocknet. Es werden ca. 62 kg (88,6 5 d. Th.) Reinguanin erhalten, welches der gültigen Prüfungsvorschrift entspricht.

### Beispiel 5

### Cyclosierung in Formamid

In einer 750 ml Rührapparatur werden 420 ml Formamid (Gehalt > 99 %, Wassergehalt < 0,3 %) auf ca. 195°C erhitzt und 169,1 g FTAHP eingetragen. Nach 1 h Rühren unter Rückfluß bei 190-195°C wird auf ca. 120°C abgekühlt, der Niederschlag abgesaugt, gründlich mit Methanol gewaschen und zur Gewichtskonstanz getrocknet. Rohausbeute: 148,0 g, Gehalt (HPLC) 96,8 %. Zur Reinigung werden 35 g des Rohguanins in 170 ml Wasser unter Zusatz von 30 ml 45%iger Natronlauge gelöst und bei Raumtemperatur 3mal mit jeweils 3,5 g Aktivkohle behandelt. Aus der gereinigten Lösung wird das Reinguanin mit verdünnter Schwefelsäure bei erhöhter Temperatur gefällt, abgesaugt, gründlich mit Wasser und Methanol gewaschen und getrocknet. Die Farbe des Reinproduktes wird anhand der Transparenz einer 5%igen Lösung in 2n Natronlauge (4 cm Küvette) bewertet: Transparenz 38 %.

### Beispiel 6

### Cyclisierung in Formamid mit Wasserzusatz

Es wird wie in Beispiel 5 verfahren, dem eingesetzten Formamid werden jedoch 15 ml Wasser zugesetzt. Ausbeute 148,2 g, Gehalt (HPLC) 98,2 %, Transparenz des Reinguanins 59 %.

### Beispiel 7

### Cyclisierung in Formamid mit Ameisensäurezusatz

Es wird wie in Beispiel 5 verfahren, dem eingesetzten Formamid werden jedoch 15 ml 99%ige Ameisensäure zugesetzt.
Ausbeute 150,0 g, Gehalt (HPLC) 98,3 %, Transparenz des Reinguanins 67 %.

### Beispiel 8

### Cyclosierung in Formamid mit Zusatz von Ameisensäure und Wasser

Es wird wie in Beispiel 5 verfahren, dem eingesetzten Formamid werden jedoch 5 ml Wasser und 10 ml 85%ige Ameisensäure zugesetzt.
Ausbeute: 150,8 g, Gehalt (HPLC) 98,5 % Transparenz des Reinguanins 71 %.

## Patentansprüche

1. Verfahren zur Herstellung von Guanin aus 2,4-Diamino-5-formylamino-6-hydroxy-pyrimidin und Formamid, dadurch gekennzeichnet, daß man 2,4-Diamino-5-formylamino-6-hydroxypyrimidin bei einer Temperatur von mindestens 140°C in die mindestens doppelte Menge Formamid einträgt, wobei das Verhältnis von 2,4-Diamino-5-formylamino-6-hydroxy-pyrimidin und Formamid 1:2 bis 1:3 (Gewichtsanteile) beträgt, das Reaktionsgemisch dann für 1 bis 5 Stunden bei einer Temperatur von mindestens 140°C, aber nicht mehr als 250°C cyclisiert, anschließend abkühlt und das ausgefallene Guanin nach üblichen Methoden isoliert.

2. Verfahren nach Anspruch 1, dadurch gekennzeichnet, daß bis zu 10 % Ameisensäure und bis zu 5 % Wasser zugesetzt werden.

3. Verfahren nach einem der Ansprüche 1 bis 2, dadurch gekennzeichnet, daß die Cyclisierung bei einer Temperatur von 185 bis 195°C erfolgt.

4. Verfahren nach einem der Ansprüche 1 bis 3, dadurch gekennzeichnet, daß man das isolierte Guanin in verdünnter Natronlauge löst, ein oder mehrfach mit Aktivkohle behandelt und anschließend mit Säure wieder in Guanin überführt.

5. Verfahren nach einem der Ansprüche 1 bis 4, dadurch gekennzeichnet, daß man nach der Cyclisierung auf unterhalb 100°C abkühlt, mit ca. demselben Volumen Wasser versetzt, mit einem Alkalimetallhydroxid auf eine Alkalikonzentration von mindestens 10 Gew.-% bezogen auf das Gesamtgewicht der Reaktionsmasse einstellt und das ausfallende grobkristalline Salz isoliert.

6. Verfahren nach Anspruch 5, dadurch gekennzeichnet, daß als Alkalimetallhydroxid Natriumhydroxid verwendet wird.

## Claims

1. Process for preparing guanine from 2,4-diamino-5-formylamino-6-hydroxy-pyrimidine and formamide, characterised in that 2,4-diamino-5-formylamino-6-hydroxypyrimidine is added to at least twice the quantity of formamide at a temperature of at least 140°C, the ratio of 2,4-diamino-5-formylamino-6-hydroxypyrimidine to formamide being 1:2 to 1:3 (parts by weight), the reaction mixture is then cyclised for 1 to 5 hours at a temperature of at least 140°C but not more than 250°C, then cooled, and the guanine precipitated is isolated by conventional methods.

2. Process according to claim 1, characterised in that up to 10% formic acid and up to 5% water are added.

3. Process according to one of claims 1 and 2, characterised in that the cyclisation is carried out at a temperature of 185 to 195°C.

4. Process according to one of claims 1 to 3, characterised in that the isolated guanine is dissolved in dilute sodium hydroxide solution, treated once or several times with activated charcoal and then converted back into guanine with acid.

5. Process according to one of claims 1 to 4, characterised in that after cyclisation the reaction mixture is cooled to below 100°C, mixed with about the same volume of water, adjusted to an alkali concentration of at least 10% by weight, based on the total weight of the reaction mass, using an alkali metal hydroxide and the coarse crystalline salt precipitated is isolated.

6. Process according to claim 5, characterised in that sodium hydroxide is used as the alkali metal hydroxide.

## Revendications

1. Procédé pour la préparation de la guanine à partir de 2,4-diamino-5-formylamino-6-hydroxy-pyrimidine et de formamide, caractérisé en ce que l'on introduit la 2-4-diamino-5-formylamino-6-hydroxypyrimidine à une température d'au moins 140°C dans au moins la quantité double de formamide, le rapport entre la 2-4-diamino-5-formylamino-6-hydroxy-pyrimidine et le formamide étant de 1:2 jusqu'à 1:3 (partie en poids), le mélange réactionnel étant alors cyclisé pendant une à cinq heures à une température d'au moins 140°C mais ne dépassant pas 250°C, consécutivement il est refroidi et la guanine précipitée est isolée selon les méthodes connues.

2. Procédé selon la revendication 1, caractérisé en ce que on ajoute jusqu'à 10 % d'acide formique et jusqu'à 5 % d'eau.

3. Procédé selon l'une des revendications 1 à 2, caractérisé en ce que la cyclisation s'effectue à une température de 185 à 195°C.

4. Procédé selon l'une des revendications 1 à 3, caractérisé en ce que l'on dissout la guanine isolée dans une solution de soude diluée, ou que l'on traite une ou plusieurs fois avec du charbon actif et que l'on transforme consécutivement avec de l'acide à nouveau en guanine.

5. Procédé selon l'une des revendications 1 à 4, caractérisé en ce qu' après la cyclisation on refroidit à une température inférieure à 100°C, on ajoute approximativement le même volume d'eau, on ajuste avec un hydroxyde des métaux alcalins à une concentration alcaline d'au moins 10 % en poids rapportés au poids global de la masse réactionnelle, et l'on isole le sel cristallin grossier précipité.

6. Procédé selon la revendication 5, caractérisé en ce qu'en tant qu'hydroxyde de métaux alcalins on utilise de l'hydroxyde de sodium.
